(19) Europäisches Patentamt / European Patent Office / Office européen des brevets

(11) **EP 3 154 777 B1**

(12) **EUROPEAN PATENT SPECIFICATION**

(45) Date of publication and mention of the grant of the patent:
**14.05.2025 Bulletin 2025/20**

(21) Application number: **15731215.8**

(22) Date of filing: **12.06.2015**

(51) International Patent Classification (IPC):
**B32B 5/02** *(2006.01)*  **B32B 27/08** *(2006.01)*
**B32B 27/12** *(2006.01)*  **B32B 27/20** *(2006.01)*
**B32B 27/32** *(2006.01)*  **B32B 3/26** *(2006.01)*
**A61F 13/15** *(2006.01)*

(52) Cooperative Patent Classification (CPC):
**A61F 13/51462; A61F 13/15; A61F 13/5148;
B32B 5/022; B32B 7/04; B32B 27/08; B32B 27/12;
B32B 27/205; B32B 27/32;** B32B 3/26;
B32B 2250/246; B32B 2264/102; B32B 2264/104;
B32B 2270/00; B32B 2307/102;  (Cont.)

(86) International application number:
**PCT/US2015/035456**

(87) International publication number:
**WO 2015/191944 (17.12.2015 Gazette 2015/50)**

(54) **MULTILAYER FILMS, AND ARTICLES MADE THEREFROM**

MEHRSCHICHTFOLIEN UND DARAUS HERGESTELLTE ARTIKEL

FILMS MULTICOUCHES ET ARTICLES FABRIQUÉS À PARTIR DE CEUX-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.06.2014 US 201462011227 P**

(43) Date of publication of application:
**19.04.2017 Bulletin 2017/16**

(73) Proprietors:
• **Dow Global Technologies LLC
Midland, MI 48674 (US)**
• **Dow Quimica Mexicana S.A.de C.V.
11000 Mexico Distrito Federal (MX)**

(72) Inventors:
• **ARTEAGA LARIOS, Fabricio
06500 Mexico City (MX)**
• **DE GROOT, Jacqueline A.
Freeport, Texas 77541 (US)**
• **KALIHARI, Vivek
Freeport, Texas 77541 (US)**

(74) Representative: **Boult Wade Tennant LLP
Salisbury Square House
8 Salisbury Square
London EC4Y 8AP (GB)**

(56) References cited:
WO-A1-00/06381    WO-A1-00/38915
WO-A1-01/83599    WO-A1-03/020513
WO-A1-98/02610    WO-A1-98/58799
WO-A1-99/14047    WO-A1-99/23139
US-A1- 2002 187 304    US-A1- 2003 106 560
US-A1- 2004 122 388    US-A1- 2005 118 435
US-A1- 2008 108 268

(52) Cooperative Patent Classification (CPC): (Cont.)
B32B 2307/514; B32B 2307/558; B32B 2307/72;
B32B 2307/724; B32B 2535/00; B32B 2555/00;
B32B 2555/02

**Description**

FIELD

Embodiments of the present disclosure generally relate to multilayer films and applications of the multilayer films to make articles, such as, for example, ultrasonically-bonded laminates. In particular, this disclosure relates to breathable multilayer films.

BACKGROUND

Cloth-like backsheets have become increasingly desirable for use in hygiene absorbent products, such as, for example, diapers, adult incontinence products, and feminine hygiene articles, in order to provide good haptics, such as, softness, and low noise, while still offering sufficient barrier properties to perform its primary function of containing fluids. Cloth-like backsheets typically include a nonwoven substrate and a film laminated together, and depending on the lamination technology involved, the haptics of the backsheet can vary. Several different lamination technologies exist for joining films and nonwovens, and can include, for example, extrusion coating, hot melt adhesive, solvent-less adhesives, and ultrasonic bonding. Each lamination technique has its own particularities. In recent years, ultrasonic bonding has become an emerging lamination technology for use in producing backsheets; however, it is not without its challenges. One major challenge observed when using ultrasonic bonding is that where different types of materials are used for the nonwoven substrate and the film, (e.g., a polyethylene-based film laminated to a polypropylene nonwoven substrate), adhesion is adversely affected often resulting in a poor bond between the two. In addition, pinholes can result which can destroy the liquid barrier functionality of the backsheet.

US 2003/106560 A1 relates to a single-use, disposable absorbent laminate containing one or more layers of hydrophilic meltspun material bonded to a breathable film. US 20081108268 A1 relates to a film and a film/nonwoven laminate fabric, having breathability to water vapor and barrier to the passage of liquid and viruses.

Accordingly, alternative multilayer films that can provide good adhesion to a nonwoven polypropylene substrate, and articles comprising multilayer films having good haptics, such as softness, and low noise, as well as, reduced pinholes are desired. It is also desirable to have multilayer films that are breathable because breathable films reduce moisture retention and this facilitates comfort.

SUMMARY

The films of the invention comprise a core layer and two skin layers, wherein the core layer is positioned between the two skin layers, wherein the core layer comprises a polyethylene polymer blend, the polyethylene polymer blend comprising at least 40% by weight of the polyethylene polymer blend, of an ethylene-based polymer having a density of 0.900-0.940 g/cc and a melt index of 0.7-6 g/10 min, wherein the polyethylene polymer blend has an overall density of about 0.910-0.950 g/cc and a melt index of about 0.7-6 g/ 10 min, and wherein the polyethylene polymer blend comprises from 0 to 15 %, by weight of the polyethylene polymer blend, of a low density polyethylene. Each skin layer independently comprises a propylene-based polymer; wherein the propylene-based polymer comprises a propylene homopolymer, wherein each skin layer independently comprises at least 55 wt.% of the propylene homopolymer, wherein the skin layers are not non-wovens; and wherein the multilayer film further comprises a filler, the filler being present in one or more layers of the multilayer film. In an embodiment, every layer of the multilayer film contains an effective amount of the filler that renders the film breathable upon being subjected to stretching. After the filler is incorporated into the respective layers, the layers are subjected to stretching (also termed elongation) thus rendering the multilayer films breathable. The elongation may be unaxial or biaxial.

In some embodiments, one or more layers of the multilayer film comprise the filler. The filler comprises a metal oxide, a metal hydroxide or a combination thereof. The filler may be porous or non-porous. Calcium carbonate is the preferred filler.

In some embodiments, the breathable films are produced by blending the filler with the desired polymers; extruding the respective core and skin layers; bonding the respective layers to one another to form a film; and heating and permanently elongating the film to render the film microporous.

In some embodiments herein, the polyethylene polymer blend further comprises a low density polyethylene having a density of about 0.915-0.930 g/cc and a melt index of about 1-15 g/10 min. In some embodiments herein, the polyethylene polymer blend further comprises a medium or high density polyethylene having a density of about 0.930-0.965 g/cc and a melt index of about 1-10 g/10 min. In some embodiments herein, the polyethylene polymer blend comprises 10% to 30%, by weight of the polyethylene polymer blend, of the medium or high density polyethylene. In some embodiments herein, the polyethylene polymer blend further comprises 5% to 15%, by weight of the polyethylene polymer blend, of a low density polyethylene having a density of about 0.915-0.930 g/cc and a melt index of about 1-15 g/10 min, and 10% to 25%, by weight of the polyethylene polymer blend, of a medium or high density polyethylene having a density of about 0.930-0.965 g/cc and a melt index of about 1-10 g/10 min.

In some embodiments herein, the propylene-based polymer comprises a polypropylene polymer blend that further comprises a low density polyethylene having a density of about 0.915-0.930 g/cc and a melt index of about 1-15 g/10 min.

In some embodiments herein, the core layer comprises from about 50% to about 80% of the overall film thickness. In some embodiments herein, the two skin layers have an equal thickness. In some embodiments, the two skin layers may not have equal thicknesses. In some embodiments herein, each skin layer further comprises a compatibilizer agent capable of compatibilizing blends of polyethylene and polypropylene polymers. In some embodiments herein, the compatibilizer agent comprises polyolefin plastomers or polyolefin elastomers.

In some embodiments herein, the film has a basis weight of between about 5-40 gsm. In some embodiments herein, the film exhibits at least one of the following properties: a spencer dart impact strength of greater than 140 g, a 2% secant modulus of greater than about 16,000 psi in the MD and greater than 16,000 psi in the CD, a stress at break in the cross-direction of greater than about 1,700 psi, and in the machine direction of greater than about 2,000 psi, or a puncture resistance greater than about 15 ft·lbf/in$^3$. In some embodiments herein, the film exhibits at least one of the following properties: a softness value difference of less than 5%, when compared to a 100% polyethylene film having a 2% secant modulus greater than about 16,000 psi in the machine direction; or a noise value of less than 0.5 dB between a frequency band of 1,000 Hz and 5,000 Hz.

Also disclosed in embodiments herein are ultrasonically bonded laminates. The laminates comprise a multilayer film according to one or more embodiments herein, and a nonwoven substrate at least partially ultrasonically bonded to the multilayer film. In some embodiments herein, the nonwoven substrate is made from a propylene-based material. In some embodiments herein, the laminate exhibits at least one of the following properties: a peel force value of greater than about 0.3 lbf/inch or a hydrostatic pressure above 20 mbar. In some embodiments, the peel strength of the laminate from the nonwoven substrate is at least 0.3 lbf/inch, preferably at least 0.5 lbf/inch, preferably at least 1 lbf/inch, preferably at least 1.5 lbf/inch, and more preferably at least 2 lbf/inch. The peel strength can be at least up to 5 lbf/inch.

Additional features and advantages of the embodiments will be set forth in the detailed description which follows, and in part will be readily apparent to those skilled in the art from that description or recognized by practicing the embodiments described herein, including the detailed description which follows, the claims, as well as the appended drawings.

It is to be understood that both the foregoing and the following description describe various embodiments and are intended to provide an overview or framework for understanding the nature and character of the claimed subject matter. The accompanying drawings are included to provide a further understanding of the various embodiments, and are incorporated into and constitute a part of this specification. The drawings illustrate the various embodiments described herein, and together with the description serve to explain the principles and operations of the claimed subject matter.

Conversion units used in this specification:

1mil= 0.025 mm
1lbf/inch= 175.2 N/m
1000 psi= 6.89 MPa
1ft.lbf/in$^3$= 82.7 kJ/m$^3$

BRIEF DESCRIPTION OF THE DRAWINGS

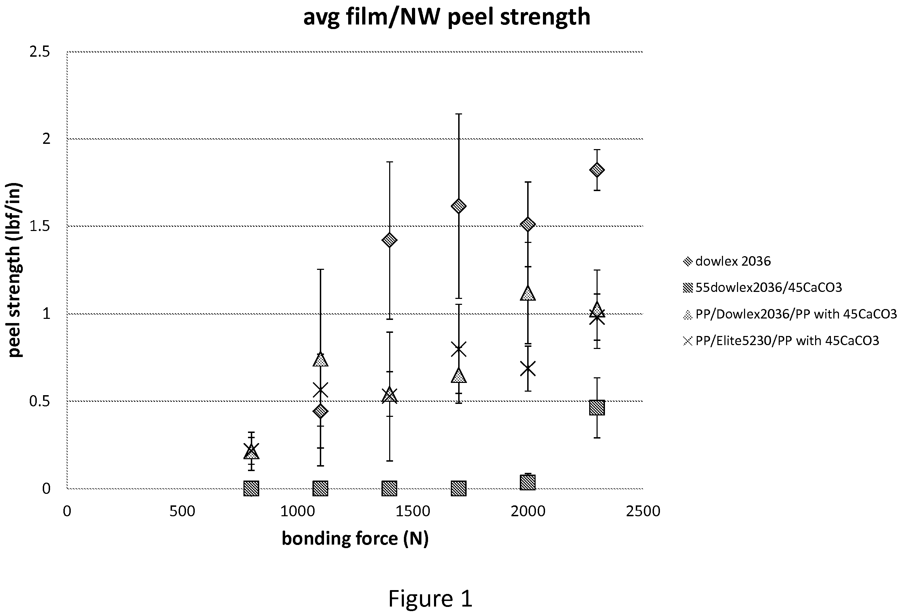

Figure 1 graphically depicts the peel strength and the bonding force for a multilayer film according to one of more embodiments shown or described herein in comparison to comparative films.

DETAILED DESCRIPTION

Reference will now be made in detail to embodiments of breathable multilayer films (hereinafter called "multilayer films") and ultrasonically-bonded laminates, examples of which are further described in the accompanying figures. The multilayer films may be used to produce cloth-like backsheets. It is noted, however, that this is merely an illustrative implementation of the embodiments disclosed herein. The embodiments are applicable to other technologies that are susceptible to similar problems as those discussed above. For example, multilayer films used to produce cloth-like wipes, face masks, surgical gowns, tissues, bandages and wound dressings are clearly within the purview of the present embodiments. As used herein, "multilayer film" refers to a film having two or more layers that are at least partially contiguous and preferably, but optionally, coextensive.

The multilayer films comprise a core layer and two skin layers. The core layer is positioned between the two skin layers. It is to be noted that the skin layers of the multilayer films are not non-wovens. In an embodiment, the core layer may comprise more than two layers or more than three layers or more than five layers. As stated above, one or more of the layers of the multilayer film are breathable. In some embodiments, all layers of the multilayer film are breathable. Breathability is conferred to one or more layers of the multilayer film by adding a filler to the layers and then subjecting the one or more layers to stretching. The stretching of the layers is called activation of the layers and it produces micro-porosity in the films. The micro-porosity renders the film breathable. The filler is an inorganic material. In an exemplary embodiment, the filler is calcium carbonate.

In some embodiments, the multilayer films may comprise one or more additional layers, such as structural, barrier, or tie layers, positioned between the core layer and each skin layer. Various materials can be used for these layers and can include polypropylene-based plastomers or elastomers, ethylene/vinyl alcohol (EVOH) copolymers, polyvinylidene chloride (PVDC), polyethylene terepthalate (PET), oriented polypropylene (OPP), ethylene/vinyl acetate (EVA) copolymers, ethylene/acrylic acid (EAA) copolymers, ethylene/methacrylic acid (EMAA) copolymers, polyacrylic imides, butyl acrylates, peroxides (such as peroxypolymers, e.g., peroxyolefins), silanes (e.g., epoxysilanes), reactive polystyrenes, chlorinated polyethylene, olefin block copolymers, propylene copolymers, propylene-ethylene copolymers, ULDPE, LLDPE, HDPE, MDPE, LMDPE, LDPE, ionomers, and graft-modified polymers (e.g., maleic anhydride grafted polyethylene).

The core layer comprises a polyethylene polymer blend, the polyethylene polymer blend comprising an ethylene-based polymer. Each skin layer independently comprises a propylene-based polymer. The propylene-based polymer is a propylene homopolymer.

In embodiments herein, the multilayer films may be polyethylene-based. As used herein in reference to multilayer films, "polyethylene-based" means that the multilayer films are primarily (i.e., greater than 50%, by total weight of the multilayer film) comprised of polyethylene resin. "Polyethylene" refers to a homopolymer of ethylene or a copolymer of ethylene with one or more comonomers with a majority of its polymer units derived from ethylene. Also disclosed herein are ultrasonically-bonded laminates comprising the multilayer films.

The thickness ratio of both skin layers to the core layer can be a ratio suitable to impart good ultrasonic bonding properties to the film. In some embodiments, the thickness ratio of both skin layers to the core layer may be 1:10 to 1:1. In other embodiments, the thickness ratio of both skin layers to the core layer may be 1:5 to 1:1. In further embodiments, the thickness ratio of both skin layers to the core layer may be 1:4 to 1:2. The thickness ratio of both skin layers to the core layer can also be captured by percentages. For example, in some embodiments, the core layer comprises greater than 50% to 90% of the overall film thickness. In other embodiments, the core layer comprises from 60% to 85% of the overall film thickness. In further embodiments, the core layer comprises from 65% to 80% of the overall film thickness. In embodiments herein, the two skin layers may have an equal thickness, or alternatively, may have an unequal thickness.

Core Layer

The core layer comprises a polyethylene polymer blend. As used herein, "polyethylene polymer blend" refers to a mixture of two or more polyethylene polymers. The polyethylene polymer blend may be immiscible, miscible, or compatible. Each of the two or more polyethylene polymers comprise greater than 50%, by weight, of its units derived from an ethylene monomer. This may include polyethylene homopolymers or copolymers (meaning units derived from two or more comonomers). In embodiments herein, the polyethylene polymer blend comprises at least 30 wt.% of the core layer. In some embodiments, the polyethylene polymer blend may comprise at least 35 wt.% of the core layer, at least 40 wt.% of the core layer, at least 50 wt.% of the core layer, or at least 55 wt.% of the core layer.

In embodiments herein, the polyethylene polymer blend has an overall density of 0.910-0.950 g/cc. All individual values and subranges from 0.910-0.950 g/cc are included and disclosed herein. For example, in some embodiments, the polyethylene polymer blend has an overall density of 0.915 - 0.930 g/cc. In other embodiments, the polyethylene polymer blend has an overall density of 0.920 - 0.930 g/cc. In further embodiments, the polyethylene polymer blend has an overall density of 0.920 - 0.940 g/cc. Densities disclosed herein for ethylene-based polymers are determined according to ASTM D-792.

The polyethylene polymer blend has an overall melt index of about 0.7-6 g/ 10 min. All individual values and subranges from 0.7-6 g/10 min are included and disclosed herein. For example, in some embodiments, the polyethylene polymer blend has a melt index of 2-6 g/10 min. In other embodiments, the polyethylene polymer blend has a melt index of 3-6 g/10 min. In further embodiments, the polyethylene polymer blend has a melt index of 4-6 g/10 min. Melt index, or I2, for ethylene-based polymers is determined according to ASTM D1238 at 190° C, 2.16 kg.

The polyethylene polymer blend comprises at least 40%, by weight of the polyethylene polymer blend, of an ethylene-based polymer. In some embodiments, the polyethylene polymer blend comprises at least 50%, at least 55%, at least 60%, at least 65%, at least 70%, at least 75%, at least 80%, or at least 85%, by weight of the polyethylene polymer blend, of an ethylene-based polymer. The ethylene-based polymer has a polymer backbone that lacks measurable or demonstrable long chain branches. As used herein, "long chain branching" means branches having a chain length greater than that of any short chain branches, which are a result of comonomer incorporation. The long chain branch can be about the same length or as long as the length of the polymer backbone. In some embodiments, the ethylene-based polymer is substituted with an average of from 0.01 long chain branches/1000 carbons to 3 long chain branches/1000 carbons, from 0.01 long chain branches/1000 carbons to 1 long chain branches/1000 carbons, from 0.05 long chain branches/1000 carbons to 1 long chain branches/1000 carbons. In other embodiments, the ethylene-based polymer is substituted with an average of less than 1 long chain branches/1000 carbons, less than 0.5 long chain branches/1000 carbons, or less than 0.05 long chain branches/1000 carbons, or less than 0.01 long chain branches/1000 carbons. Long chain branching (LCB) can be determined by conventional techniques known in the industry, such as 13C nuclear magnetic resonance (13C NMR) spectroscopy, and can be quantified using, for example, the method of Randall (Rev. Macromol. Chem. Phys., C29 (2 & 3), p. 285-297). Two other methods that may be used include gel permeation chromatography coupled with a low angle laser light scattering detector (GPC-LALLS), and gel permeation chromatography coupled with a differential viscometer detector (GPC-DV). The use of these techniques for long chain branch detection, and the underlying theories, have been well documented in the literature. See, for example, Zimm, B. H. and Stockmayer, W. H., J. Chem. Phys., 17, 1301 (1949) and Rudin A., Modern Methods of Polymer Characterization, John Wiley & Sons, New York (1991), pp. 103-112.

In some embodiments, the ethylene-based polymer may be homogeneously branched or heterogeneously branched and/or unimodal or multimodal (e.g., bimodal) polyethylene. The ethylene-based polymer comprises ethylene homopolymers, copolymers or ethylene-derived units and at least one type of comonomer and blends thereof. Examples of suitable comonomers may include $\alpha$-olefins. Suitable $\alpha$-olefins may include those containing 3 to 20 carbon atoms (C3-C20). For example, the $\alpha$-olefin may be a C4-C20 $\alpha$-olefin, a C4-C12 $\alpha$-olefin, a C3-C10 $\alpha$-olefin, a C3-C8 $\alpha$-olefin, a C4-C8 $\alpha$-olefin, or a C6-C8 $\alpha$-olefin. In some embodiments, the ethylene-based polymer is an ethylene/$\alpha$-olefin copolymer, wherein the $\alpha$-olefin is selected from the group consisting of propylene, 1-butene, 1-pentene, 1-hexene, 4-methyl-1-pentene, 1-heptene, 1-octene, 1-nonene and 1-decene. In other embodiments, the ethylene-based polymer is an ethylene/$\alpha$-olefin copolymer, wherein the $\alpha$-olefin is selected from the group consisting of propylene, 1-butene, 1-hexene, and 1-octene. In further embodiments, the ethylene-based polymer is an ethylene/$\alpha$-olefin copolymer, wherein the $\alpha$-olefin is selected from the group consisting of 1-hexene and 1-octene. In even further embodiments, the ethylene-based polymer is an ethylene/$\alpha$-olefin copolymer, wherein the $\alpha$-olefin is 1-octene. In even further embodiments, the ethylene-based polymer is a substantially linear ethylene/$\alpha$-olefin copolymer, wherein the $\alpha$-olefin is 1-octene. In some embodiments, the ethylene-based polymer is an ethylene/$\alpha$-olefin copolymer, wherein the $\alpha$-olefin is 1-butene.

The ethylene/$\alpha$-olefin copolymers may comprise at least 50%, for example, at least 60%, at least 70%, at least 80%, at least 90%, at least 92%, at least 95%, at least 97%, by weight, of the units derived from ethylene; and less than 30%, for example, less than 25%, less than 20%, less than 15%, less than 10%, less than 5%, less than 3%, by weight, of units derived from one or more $\alpha$-olefin comonomers.

Other examples of suitable ethylene-based polymers include substantially linear ethylene polymers, which are further defined in U.S. Pat. No. 5,272,236, U.S. Pat. No. 5,278,272, U.S. Pat. No. 5,582,923 and U.S. Pat. No. 5,733,155; homogeneously branched linear ethylene polymer compositions, such as those in U.S. Pat. No. 3,645,992; heterogeneously branched ethylene polymers, such as those prepared according to the process disclosed in U.S. Pat. No. 4,076,698; and/or blends thereof (such as those disclosed in U.S. Pat. No. 3,914,342 or U.S. Pat. No. 5,854,045). In some embodiments, the ethylene-based polymer may be a linear low density (LLDPE) polymer or substantially LLDPE polymer, and may include AFFINITY™ resins, ELITE™ resins, or ATTANE™ resins sold by The Dow Chemical Company, including ELITE™ 5230G resin, ATTANE™ 4404 resin, ATTANE™ 4202 resin, or AFFINITY™ 1840 resin; DOWLEX™ 2247 resin; or EXCEED™ resins sold by Exxon Mobil Corporation, including EXCEED™ 3518 resin or EXCEED™ 4518 resin; and EXACT™ resins sold by Exxon Mobil Corporation, including EXACT™ 3024.

The ethylene-based polymer can be made via gas-phase, solution-phase, or slurry polymerization processes, or any combination thereof, using any type of reactor or reactor configuration known in the art, e.g., fluidized bed gas phase reactors, loop reactors, stirred tank reactors, batch reactors in parallel, series, and/or any combinations thereof. In some embodiments, gas or slurry phase reactors are used. Suitable ethylene-based polymers may be produced according to the processes described at pages 15-17 and 20-22 in WO 2005/111291 A1. The catalysts used to make the ethylene-based polymer described herein may include Ziegler-Natta, metallocene, constrained geometry, or single site catalysts. In some embodiments, the ethylene-based polymer may be a LLDPE, such as, a znLLDPE, which refers to linear polyethylene made using Ziegler-Natta catalysts, a uLLDPE or "ultra linear low density polyethylene," which may include linear polyethylenes made using Ziegler-Natta catalysts, or a mLLDPE, which refers to LLDPE made using metallocene or constrained geometry catalyzed polyethylene. The ethylene-based polymer has a density of 0.900 - 0.940 g/cc. All individual values and subranges from 0.900 - 0.940 g/cc are included and disclosed herein. For example, in some embodiments, the ethylene-based polymer has a density of 0.910 - 0.925 g/cc. In other embodiments, the ethylene-based polymer has a density of 0.900 - 0.920 g/cc. In further embodiments, the ethylene-based polymer has a density of 0.910 - 0.920 g/cc. Densities disclosed herein are determined according to ASTM D-792.

The ethylene-based polymer has a melt index, or I2, of 0.7-6 g/10 min. All individual values and subranges from 0.7-6 g/10 min are included and disclosed herein. For example, in some embodiments, the ethylene-based polymer has a melt index of 2-5 g/10 min. In other embodiments, the ethylene-based polymer has a melt index of 2.5-4.5 g/10 min. Melt index, or I2, for ethylene-based polymers is determined according to ASTM D1238 at 190° C, 2.16 kg.

The ethylene-based polymer disclosed herein may have a puncture resistance of greater than 100 ft·lbf/in³. All individual values and subranges of greater than 100 ft·lbf/in³ are included and disclosed herein. For example, in some embodiments, the ethylene-based polymer has a puncture resistance of greater than 125 ft·lbf/in³. In other embodiments, the ethylene-based polymer has a puncture resistance of greater than 150 ft·lbf/in³. In further embodiments, the ethylene-based polymer has a puncture resistance of greater than 175 ft·lbf/in³. In even further embodiments, the ethylene-based polymer has a puncture resistance of greater than 200 ft·lbf/in³. Puncture resistance may be measured as described below in the test methods.

The ethylene-based polymer disclosed herein may have a spencer dart impact of greater than 100 g. All individual values and subranges of greater than 100 g are included and disclosed herein. For example, in some embodiments, the ethylene-based polymer has a spencer dart impact of greater than 115 g. In other embodiments, the ethylene-based polymer has a spencer dart impact of greater than 125 g. In further embodiments, the ethylene-based polymer has a spencer dart impact of greater than 135 g. In even further embodiments, the ethylene-based polymer has a spencer dart impact of greater than 150 g. Spencer dart impact may be measured as described below in the test methods.

In one embodiment, the ethylene-based polymer is a Ziegler-Natta catalyzed ethylene and octene copolymer, having a density from about 0.900 g/cc to about 0.940 g/cc. In another embodiment, the ethylene-based polymer is a single-site catalyzed LLDPE that is multimodal.

In embodiments herein, the polyethylene polymer blend may further comprise from 0 to 30%, by weight of the polyethylene polymer blend, of a low density polyethylene (LDPE). All individual values and subranges from 0 to 30% are included and disclosed herein. For example, in some embodiments, the polymer blend may further comprise from 5 to 20%, by weight of the polyethylene polymer blend, of a low density polyethylene. In other embodiments, the polymer blend may further comprise from 5 to 15%, by weight of the polyethylene polymer blend, of a low density polyethylene. In further, embodiments, the polymer blend may further comprise from 10 to 15%, by weight of the polyethylene polymer blend, of a low density polyethylene.

In embodiments herein, the LDPE present in the polyethylene polymer blend may have a density of about 0.915-0.930 g/cc. All individual values and subranges from 0.915-0.930 g/cc are included and disclosed herein. For example, in some embodiments, the LDPE has a density of 0.915 - 0.925 g/cc. In other embodiments, the LDPE has a density of 0.915 - 0.920 g/cc. In embodiments herein, the LDPE present in the polyethylene polymer blend has a melt index of 0.2-15 g/10 min. All individual values and subranges from 0.2-15 g/10 min are included and disclosed herein. For example, in some embodiments, the LDPE has a melt index of 1-12 g/10 min. In other embodiments, the LDPE has a melt index of 5-10 g/10 min.

The LDPE present in the polyethylene polymer blend may have a melt strength of greater than 5 cN. All individual values and subranges of greater than 5cN are included and disclosed herein. For example, in some embodiments, the LDPE has a melt strength of from 6-15 cN. In other embodiments, the LDPE has a melt strength of from 6-14 cN. In further embodiments, the LDPE has a melt strength of from 6-12 cN. In further embodiments, the LDPE has a melt strength of from 6-10 cN. In even further embodiments, the LDPE has a melt strength of from 6-18 cN.

The LDPE may include branched interpolymers that are partly or entirely homopolymerized or copolymerized in autoclave or tubular reactors at pressures above 14,500 psi (100 MPa) with the use of free-radical initiators, such as peroxides (see, for example U.S. Pat. No. 4,599,392). Examples of suitable LDPEs may include, but are not limited to, ethylene homopolymers, and high pressure copolymers, including ethylene interpolymerized with, for example, vinyl acetate, ethyl acrylate, butyl acrylate, acrylic acid, methacrylic acid, carbon monoxide, or combinations thereof. Exemplary LDPE resins may include resins sold by The Dow Chemical Company, such as, LDPE 722, LDPE 5004 and LDPE 621i. Other exemplary LDPE resins are described in WO 2005/023912.

In embodiments herein, the polyethylene polymer blend may further comprise from 0 to 30%, by weight of the polyethylene polymer blend, of a medium density polyethylene (MDPE) or a high density polyethylene (HDPE). All individual values and subranges from 0 to 30% are included and disclosed herein. For example, in some embodiments, the polymer blend may further comprise from 5 to 25%, by weight of the polyethylene polymer blend, of a medium or high density polyethylene. In other embodiments, the polymer blend may further comprise from 15 to 25%, by weight of the polyethylene polymer blend, of a medium or high density polyethylene. In further, embodiments, the polymer blend may further comprise from 20 to 25%, by weight of the polyethylene polymer blend, of a medium or high density polyethylene.

In embodiments herein, the MDPE or HDPE that may be present in the polyethylene polymer blend may have a density of about 0.930-0.965 g/cc. All individual values and subranges from 0.930-0.965 g/cc are included and disclosed herein. For example, in some embodiments, the MDPE or HDPE has a density of 0.940 - 0.965 g/cc. In other embodiments, the MDPE or HDPE has a density of 0.940 - 0.960 g/cc. In further embodiments, the MDPE or HDPE has a density of 0.945 - 0.955 g/cc. In embodiments herein, the MDPE or HDPE that may be present in the polyethylene polymer blend has a melt index of 1-10 g/10 min. All individual values and subranges from 1-10 g/10 min are included and disclosed herein. For example, in some embodiments, the MDPE or HDPE has a melt index of 2-10 g/10 min. In other embodiments, the MDPE or HDPE has a melt index of 3-8 g/10 min. In further embodiments, the MDPE or HDPE has a melt index of 5-7 g/10 min.

The MDPE or HDPE may be produced in various commercially available continuous reaction processes, particularly, those comprising two or more individual reactors in series or parallel using slurry, solution or gas phase process technology or hybrid reaction systems (e.g. combination of slurry and gas phase reactor). Exemplary processes may be found in U.S. Patent 4,076,698, which is herein incorporated by reference. Alternatively, the MDPE or HDPE polymers may also be produced by offline blending of 2 or more different polyethylene resins. For example, in some embodiments, a conventional mono-modal Ziegler-Natta MDPE or HDPE may be blended with a multi-modal Ziegler-Natta MDPE or HDPE. It is contemplated, however, that the various HDPE polymers can be produced with alternative catalyst systems, such as, metallocene, post-metallocene or chromium-based catalysts. Exemplary MDPE or HDPE resins may include resins sold by The Dow Chemical Company under the trade name HDPE 5962B, DMDA 8007 NT 7, AGILITY™ 6047G and DOWLEX™ 2027G.

In some embodiments, the polyethylene polymer blend comprises at least 70%, by weight of a polyethylene polymer blend, of an ethylene-based polymer having a density of 0.900-0.940 g/cc and a melt index of 0.7-6 g/10 min, and further comprises 5% to 15%, by weight of the polyethylene polymer blend, of a LDPE having a density of about 0.915-0.930 g/cc and a melt index of about 0.2-15 g/10 min. In other embodiments, the polyethylene polymer blend comprises at least 70%, by weight of the polyethylene polymer blend, of an ethylene-based polymer having a density of 0.900-0.940 g/cc and a melt index of 0.7-6 g/10 min, and further comprises 15% to 25%, by weight of the polyethylene polymer blend, of a medium or high density polyethylene having a density of about 0.930-0.965 g/cc and a melt index of about 1-10 g/10 min. In further embodiments, the polyethylene polymer blend comprises at least 70%, by weight of the polyethylene polymer blend, of an ethylene-based polymer having a density of 0.900-0.940 g/cc and a melt index of 0.7-6 g/10 min, and further comprises 5% to 15%, by weight of the polyethylene polymer blend, of a LDPE having a density of about 0.915-0.930 g/cc and a melt index of about 0.2-15 g/10 min, and 15% to 25%, by weight of the polyethylene polymer blend, of a medium or high density polyethylene having a density of about 0.930-0.965 g/cc and a melt index of about 1-10 g/10 min. Of course, it should be understood that the foregoing amounts, density ranges, and melt index ranges are exemplary, and other amounts, density ranges, and melt index ranges as previously described herein, may be incorporated into various embodiments herein.

In embodiments herein, the polyethylene polymer blend may be formed by a variety of methods. For example, it may be made by blending or mixing the polymer components together. Blending or mixing can be accomplished by any suitable mixing means known in the art, including melt or dry/physical blending of the individual components. Alternatively, the polyethylene polymer blend may be made in a single reactor or a multiple reactor configuration, where the multiple reactors may be arranged in series or parallel, and where each polymerization takes place in solution, in slurry, or in the gas phase. It should be understood that other suitable methods for blending or mixing the polymer components together may be utilized.

The core layer may optionally comprise one or more additives. Such additives may include, but are not limited to, antioxidants (e.g., hindered phenolics, such as, IRGANOX® 1010 or IRGANOX® 1076, supplied by Ciba Geigy), phosphites (e.g., IRGAFOS® 168, also supplied by Ciba Geigy), cling additives (e.g., PIB (polyisobutylene)), Standostab PEPQ™ (supplied by Sandoz), pigments, colorants, fillers (e.g., calcium carbonate, talc, mica, kaolin, perlite, diatomaceous earth, dolomite, magnesium carbonate, calcium sulfate, barium sulfate, glass beads, polymeric beads, ceramic beads, natural and synthetic silica, aluminum trihydroxide, magnesium trihydroxide, wollastonite, whiskers, wood flour, lignine, starch), TiO2, anti-stat additives, flame retardants, biocides, antimicrobial agents, and clarifiers/nucleators (e.g., HYPERFORM™ HPN-20E, MILLAD™ 3988, MILLAD™ NX 8000, available from Milliken Chemical). The one or more additives can be included in the polyethylene polymer blend at levels typically used in the art to achieve their desired purpose. In some examples, the one or more additives are included in amounts ranging from 0-10 wt.% of the polyethylene polymer blend, 0-5 wt.% of the polyethylene polymer blend, 0.001-5 wt.% of the polyethylene polymer blend, 0.001-3 wt.% of the polyethylene polymer blend, 0.05-3 wt.% of the polyethylene polymer blend, or 0.05-2 wt.% of the polyethylene polymer blend.

In an embodiment, the core layer may comprise at least 30 wt% of a filler that confers breathability to the multilayer film. This is discussed in detail later.

Skin Layers

Each skin layer independently comprises a propylene-based polymer. The skin layers are not non-wovens. The propylene-based polymer is a propylene homopolymer.

The propylene homopolymer may be isotactic, atactic or syndiotactic. In some embodiments, the propylene homopolymer is isotactic. Each skin layer comprises at least 55 wt.% of the propylene homopolymer.

Suitable polypropylenes are formed by means within the skill in the art, for example, using Ziegler-Natta catalysts, a single-site catalysts (metallocene or constrained geometry), or non-metallocene, metal-centered, heteroaryl ligand catalysts.

In embodiments herein, the propylene-based polymer has a melt flow rate (MFR) from 0.1 g/10 min to 100 g/10 min. All individual values and subranges from 0.1 g/10 min to 100 g/10 min are included and disclosed herein. For example, in some embodiments, the propylene-based polymer has a melt flow rate from 1 g/10 min to 75 g/10 min, from 2 g/10 min to 50 g/10 min, from 10 g/10 min to 45 g/10 min, or from 15 g/10 min to 40 g/10 min, as measured in accordance with ASTM D1238 (230°C, 2.16 kg). In embodiments herein, the propylene-based polymer has a density of 0.890 to 0.920 g/cc. All individual values and subranges from 0.890 to 0.920 g/cc are included and disclosed herein. For example, in some embodiments, propylene-based polymer has a density of 0.900 to 0.920 g/cc, or from 0.89 to 0.915 g/cc. The density may be determined according to ASTM D-792.

The propylene-based polymer may have a 2% secant modulus of greater than 15,000 psi. The 2% secant modulus is an average of the secant modulus in the machine direction (MD) and the cross direction (CD), and may be calculated as follows:

$$2\%\ secant\ modulus = \frac{(2\%\ secant\ modulus\,(MD) + 2\%\ secant\ modulus\ (CD))}{2}$$

All individual values and subranges greater than 15,000 psi are included and disclosed herein. For example, in some embodiments, the propylene-based polymer has a 2% secant modulus of greater than 17,500 psi. In other embodiments, the propylene-based polymer has a 2% secant modulus of greater than 20,000 psi. In further embodiments, the propylene-based polymer has a 2% secant modulus of greater than 27,500 psi. In even further embodiments, the propylene-based polymer has a 2% secant modulus of greater than 35,000 psi. In even further embodiments, the propylene-based polymer has a 2% secant modulus of from 15,000 psi to 50,000 psi. In even further embodiments, the propylene-based polymer has a 2% secant modulus of from 25,000 psi to 45,000 psi. In even further embodiments, the propylene-based polymer has a 2% secant modulus of from 30,000 psi to 45,000 psi. The 2% secant modulus may be determined according to ASTM 882.

In some embodiments herein, the skin layer may further comprise a low density polyethylene (LDPE). LDPE has a density of about 0.915-0.930 g/cc. All individual values and subranges from 0.915-0.930 g/cc are included and disclosed herein. For example, in some embodiments, the LDPE has a density of 0.915 - 0.925 g/cc. In other embodiments, the LDPE has a density of 0.915 - 0.920 g/cc. In embodiments herein, the LDPE present in the skin layers has a melt index of 1-15 g/10 min. All individual values and subranges from 1-15 g/10 min are included and disclosed herein. For example, in some embodiments, the LDPE has a melt index of 2-12 g/10 min. In other embodiments, the LDPE has a melt index of 5-10 g/10 min.

The LDPE may have a melt strength of greater than 5 cN. All individual values and subranges of greater than 5cN are included and disclosed herein. For example, in some embodiments, the LDPE has a melt strength of from 6-15 cN. In other embodiments, the LDPE has a melt strength of from 6-14 cN. In further embodiments, the LDPE has a melt strength of from 6-12 cN. In further embodiments, the LDPE has a melt strength of from 6-10 cN. In even further embodiments, the LDPE has a melt strength of from 6-18 cN.

LDPEs may include branched polymers that are partly or entirely homopolymerized or copolymerized in autoclave or tubular reactors at pressures above 14,500 psi (100 MPa) with the use of free-radical initiators, such as peroxides (see for example U.S. Pat. No. 4,599,392). Examples of suitable LDPEs may include, but are not limited to, ethylene homopolymers, and high pressure copolymers, including ethylene interpolymerized with, for example, vinyl acetate, ethyl acrylate, butyl acrylate, acrylic acid, methacrylic acid, carbon monoxide, or combinations thereof. Exemplary LDPE resins may include resins sold by The Dow Chemical Company, such as, LDPE 722, LDPE 5004, and LDPE 621i. Other exemplary LDPE resins are described in WO 2005/023912.

The skin layers may further comprise a compatibilizer agent capable of compatibilizing blends of polyethylene and polypropylene polymers. Suitable compatibilizer agents include olefin plastomers and elastomers, such as, ethylene-based and propylene-based copolymers available under the trade name VERSIFY™ (from The Dow Chemical Company), SURPASS™ (from Nova Chemicals), and VISTAMAXX™ (from Exxon Mobil Corporation).

Exemplary compatibilizers may include the VERSIFY™ 3401 compatibilizer (from The Dow Chemical Company) or the VISTAMAXX™ 6202 compatibilizer (from Exxon Mobil Corporation).

Each skin layer may independently comprise one or more additives. Such additives may include, but are not limited to, antioxidants (e.g., hindered phenolics, such as, IRGANOX® 1010 or IRGANOX® 1076, supplied by Ciba Geigy), phosphites (e.g., IRGAFOS® 168, also supplied by Ciba Geigy), cling additives (e.g., PIB (polyisobutylene)), Standostab PEPQ™ (supplied by Sandoz), pigments, colorants, fillers (e.g., calcium carbonate, mica, talc, kaolin, perlite, diatomaceous earth, dolomite, magnesium carbonate, calcium sulfate, barium sulfate, glass beads, polymeric beads, ceramic beads, natural and synthetic silica, aluminum trihydroxide, magnesium trihydroxide, wollastonite, whiskers, wood flour, lignine, starch), TiO2, anti-stat additives, flame retardants, slip agents, antiblock additives, biocides, antimicrobial agents, and clarifiers/nucleators (e.g., HYPERFORMTM HPN-20E, MILLAD™ 3988, MILLAD™ NX 8000, available from Milliken Chemical). The one or more additives can be included in the polypropylene polymer blend at levels typically used in the art to achieve their desired purpose. In some examples, the one or more additives are included in amounts ranging from 0-10 wt.% of the polypropylene polymer blend, 0-5 wt.% of the polypropylene polymer blend, 0.001-5 wt.% of the polypropylene polymer blend, 0.001-3 wt.% of the polypropylene polymer blend, 0.05-3 wt.% of the polypropylene polymer blend, or 0.05-2 wt.% of the polypropylene polymer blend.

In an embodiment, the core layer may comprise at least 30 wt% of a filler that confers breathability to the multilayer film. This is discussed in detail later.

Multilayer Films

The multilayer films described herein may be coextruded films. In some embodiments, the multilayer film is a coextruded film, whereby at least one of the skin layers is coextruded to the core layer. In other embodiments, the multilayer film is a coextruded film, whereby one of the skin layers (i.e., a first skin layer) is coextruded to the core layer and the other skin layer (i.e., a second skin layer) is coextruded to the core layer, and the two coextruded films are laminated together such that the core layer is positioned between the two skin layers. In further embodiments, the multilayer film is a coextruded film, whereby the skin layers are coextruded to the core layer.

Films may be made via any number of processes including cast film where the polymer is extruder through a flat die to create a flat film or blown film whereby the polymer is extruded through an annular die and creates a tube of film that can be slit to create the flat film.

In embodiments herein, the multilayer film may have a basis weight of between about 10-20 gsm. All individual values and subranges from 10-20 gsm are included and disclosed herein. For example, in some embodiments, the multilayer film may have a basis weight of between about 10-18 gsm. In other embodiments, the multilayer film may have a basis weight of between about 10-16 gsm. In further embodiments, the multilayer film may have a basis weight of between about 10-14 gsm.

In some embodiments, the multilayer films described herein may exhibit at least one of the following properties: a spencer dart impact of greater than about 160 g (or, alternatively, greater than 170 g or 180 g); a secant modulus at 2% of greater than about 16,000 psi in the MD (or alternatively, greater than 17,000 psi or 18,000 psi) and greater than 16, 000 psi in the CD (or alternatively, greater than 17,000 psi); a stress at break (also called load at break) in the cross-direction of greater than about 1,700 psi (or, alternatively, greater than about 1,800 psi or 1,900 psi), and in the machine direction of greater than about 2,000 psi (or, alternatively, greater than about 2,100 psi, 2,200 psi, or 2,300 psi); or a puncture resistance greater than about 30 ft·lbf/in3 (or, alternatively, 35 ft·lbf/in3 or 40 ft·lbf/in3). In some embodiments, the multilayer films described herein may exhibit at least one of the following properties: a softness value difference of less than 5%, when compared to a 100% polyethylene film having a 2% secant modulus greater than about 16,000 psi in the MD, or a noise value of less than 0.5 dB between a frequency band of 1,000 Hz and 5,000 Hz. The Softness Value Difference (SVD) may be calculated as follows:

$$SVD = \frac{|Softness\ Value\ (inventive\ film) - Softness\ Value\ (reference\ film)|}{Softness\ Value\ (reference\ film)}\ x\ 100\%$$

wherein the reference film is a 100% polyethylene film having a 2% secant modulus of greater than 16,000 psi. As used herein a "100% polyethylene film" refers to a film consisting of one or more polymers that contain more than 50 mole percent polymerized ethylene monomer (based on the total amount of polymerizable monomers) and, optionally, may contain at least one comonomer. Without being bound by theory, it is believed that one or more of the properties result from improved film structure and improved component amounts in each layer of the film structure such that key attributes of each material are incorporated. In particular, it is believed that incorporating particular amounts of polypropylene into the skin layers can assist in adhesion, while selecting a particular polyethylene blend in the core layer can avoid pinholes that may form between polypropylene substrates and polyethylene films, while still providing adequate strength and modulus necessary for a backsheet. It is also believed that by selecting certain polyethylene polymers for incorporation into the core and skin layers, the haptics properties, in particular, noise and softness, can be improved.

Laminates

Also described herein are ultrasonically-bonded laminates. The ultrasonically-bonded laminates comprise a multilayer film as previously described herein, and a nonwoven substrate at least partially ultrasonically bonded to the multilayer film. As used herein, "nonwoven substrates" include nonwoven webs, nonwoven fabrics and any nonwoven structure in which individual fibers or threads are interlaid, but not in a regular or repeating manner. Nonwoven substrates described herein may be formed by a variety of processes, such as, for example, air laying processes, meltblowing processes, spunbonding processes and carding processes, including bonded carded web processes. As used herein, "ultrasonic-bonding" includes ultrasonic welding.

The nonwoven web may comprise a single web, such as a spunbond web, a carded web, an airlaid web, a spunlaced web, or a meltblown web. However, because of the relative strengths and weaknesses associated with the different processes and materials used to make nonwoven fabrics, composite structures of more than one layer are often used in order to achieve a better balance of properties. Such structures are often identified by letters designating the various lays such as SM for a two layer structure consisting of a spunbond layer and a meltblown layer, SMS for a three layer structure, or more generically SXnS structures, where X can be independently a spunbond layer, a carded layer, an airlaid layer, a spunlaced layer, or a meltblown layer and n can be any number, although for practical purposes is generally less than 5. In order to maintain structural integrity of such composite structures, the layers must be bonded together. Common methods of bonding include point bonding, adhesive lamination, and other methods known to those skilled in the art. All of these structures may be used in the present invention.

The fibers which make up the nonwoven web are monocomponent fibers. It is preferred that the surface of the fiber comprise a polyethylene resin other than LDPE. The polyethylene resin can advantageously be a single site catalyzed resin (mLLDPE), or a post metallocene catalyzed LLDPE, or a Ziegler-Natta catalyzed LLDPE, or HDPE, or MDPE. If monocomponent fibers are used it is preferred that the resin used in the fiber comprise 100% linear (including "substantially linear") polyethylene.

In embodiments herein, the nonwoven substrate is made from a propylene-based material, 100% polyethylene, or polyethylene/polypropylene blends. The non-woven substrates do not include bicomponent non-woven fibers especially those having a polypropylene core with a polyethylene sheath. Examples of suitable propylene-based materials include materials that comprise a majority weight percent of polymerized propylene monomer (based on the total amount of polymerizable monomers), and optionally, one or more comonomers. This may include propylene homopolymer (i.e., a polypropylene), a propylene copolymer, or combinations thereof. The propylene copolymer may be a propylene/olefin copolymer. Nonlimiting examples of suitable olefin comonomers include ethylene, C4-C20 α-olefins, such as 1-butene, 1-pentene, 1-hexene, 4-methyl-1-pentene, 1-heptene, 1-octene, 1-decene, or 1-dodecene. In some embodiments, the propylene-based material is polypropylene homopolymer.

The nonwoven substrate may comprise one or more layers. The one or more layers may be spunbond non-woven layers (S), meltblown non-woven layers (M), wet-laid non-woven layers, air-laid non-woven layers, webs produced by any non-woven or melt spinning process. In some embodiments, the nonwoven substrate comprises at least one spunbond layer (S) and at least one meltblown layer (M). In other embodiments, the nonwoven substrate comprises at least one spunbond layer (S) and at least one meltblown layer (M). The non-woven substrate may have one of the following structures: SSS, SM, SMS, SMMS, SSMMS, or SSMMMS. The outermost spunbond layer may comprise a material selected from the group consisting of spunbond homopolymer polypropylene (hPP), spunbond heterogeneously branched polyethylene, or carded hPP.

The ultrasonically-bonded laminates described herein may exhibit at least one of the following properties: a peel force value of greater than 0.4 lbf/inch, greater than 0.8 lbf/inch, greater than 1.0 lbf/inch, greater than 1.2 lbf/inch and more preferably greater than 2.0 lbf/inch. Without being bound by theory, it is believed that incorporating particular amounts of polypropylene into the skin layers can assist in adhesion and selecting a particular polyethylene blend for the core layer can avoid or reduce pinholes during an ultrasonic bonding process between polypropylene substrates and polyethylene-based films. Having a higher level of pinholes can allow more water to pass through the laminate resulting in a lower hydrostatic pressure, while having a lower level of pinholes can result in a higher hydrostatic pressure due to less water passing through.

End Uses

The films or ultra-sonically bonded laminates described herein may be used in a variety of applications. In some embodiments, the films or laminates can be used in hygiene applications, such as diapers, training pants, and adult incontinence articles, or in other similar absorbent garment applications. In other embodiments, the films or laminates can be used in medical applications, such as medical drapes, gowns, and surgical suits, or in other similar fabric (woven or nonwoven) applications.

The films or laminates may be breathable or non-breathable. As used herein, the term "breathable" refers to a material which is permeable to water vapor. The water vapor transmission rate (WVTR) or moisture vapor transfer rate (MVTR) is measured in grams per square meter per 24 hours, and shall be considered equivalent indicators of breathability. The term "breathable" refers to a material which is permeable to water vapor having a minimum WVTR (water vapor transmission rate) of greater than about 500 $g/m^2/24$ hours. In some embodiments, the breathability is greater than about 800 $g/m^2/24$ hours. In other embodiments, the breathability is greater than about 1000 $g/m^2/24$ hours. In further embodiments, the breathability is greater than about 1200 $g/m^2/24$ hours up to a value of 2200 $g/m^2/24$ hours.

The WVTR of films or laminates, in one aspect, gives an indication of how comfortable the article would be to wear. Often, hygiene applications of breathable films or laminates desirably have higher WVTRs and films or laminates of the present invention can have WVTRs exceeding about 1,100 $g/m^2/24$ hours, 1,500 $g/m^2/24$ hours, 1,800 $g/m^2/24$ hours

or even exceeding 2,000 g/m$^2$/24 hours. A suitable technique for determining the WVTR (water vapor transmission rate) value of a film or laminate material of the invention is the test procedure standardized by INDA (Association of the Nonwoven Fabrics Industry), number IST-70.4-99, entitled "STANDARD TEST METHOD FOR WATER VAPOR TRANSMISSION RATE THROUGH NONWOVEN AND PLASTIC FILM USING A GUARD FILM AND VAPOR PRESSURE SENSOR" which is incorporated by reference herein. The INDA procedure provides for the determination of WVTR, the permeance of the film to water vapor and, for homogeneous materials, water vapor permeability coefficient.

Breathable films may be obtained by adding fillers to one or more layers of the multilayer film. The fillers are preferably inorganic materials. The fillers may be particulate like, fibrous, or include combinations of particulates with fibrous materials.

Breathable films may be obtained by adding fillers, like CaCO3, clay, silica, alumina, titania, zirconia, ceria, talc, magnesium carbonate, calcium sulfate, barium sulfate, porous glass beads, porous polymeric beads, ceramic beads, aluminum trihydroxide, magnesium trihydroxide, wollastonite whiskers, wood flour, lignin, starch, clay, or the like, or a combination thereof, to make moisture breathable films of high WVTR, which requires a post-orientation process, such as machine direction orientation or the use of inter-digitating or inter-meshing rollers, also called "ring rolling", to create cavitation around the filler particles (see, for example, WO2007/081548 or WO1998/004397, which are herein incorporated by reference). Enhanced moisture permeation in such films is a result of microporous morphology.

Such films are commonly used hygiene applications for diaper and adult incontinence backsheet films and in medical applications such as breathable but liquid impermeable surgical gowns and can yield WVTR values of greater than 500 g/m$^2$/24 hours up to 20,000 g/m$^2$/24 hours, depending upon the level of the filler and stretching, for films ranging in thickness from 0.2 to 1.5 mils thickness.

The filler is included in any one of the layers in an amount of 15 to 70 wt%, preferably 25 to 55 wt% and more preferably 30 to 50 wt%, based on the total weight of the layer. In another embodiment, the filler is added to the skin layers in an amount of 15 to 70 wt%, preferably 25 to 55 wt% and more preferably 30 to 50 wt%, based on the total weight of the skin layer. In an exemplary embodiment, the filler is calcium carbonate and is present in the skin layers in an amount of 40 to 50 wt%, based on the total weight of the layer. In an exemplary embodiment, the filler is calcium carbonate.

The multilayer film and the breathable layers therein is stretched or elongated after the incorporation of the filler. The stretching may be conducted after the bonding of the layers to one another or prior to the bonding of the layers to one another. The multilayer film may be stretched one or more time during the process of producing the multilayer film. In some embodiments, the multilayer film is stretched before and/or after producing a laminate or an article thereof. The stretching may be unaxial or biaxial.

TEST METHODS

Unless otherwise stated, the following test methods are used. All test methods are current as of the filing date of this disclosure.

Density

Densities disclosed herein for ethylene-based and propylene-based polymers are determined according to ASTM D-792.

Melt Index

Melt index, or I2, for ethylene-based polymers is determined according to ASTM D1238 at 190°C, 2.16 kg.

Melt Flow Rate

Melt Flow Rate, or MFR, for propylene-based polymers is measured in accordance with ASTM D1238 at 230°C, 2.16 kg.

Melt Strength

Melt Strength measurements are conducted on a Gottfert Rheotens 71.97 (Göettfert Inc.; Rock Hill, SC) attached to a Gottfert Rheotester 2000 capillary rheometer. A polymer melt (about 20-30 grams, pellets) is extruded through a capillary die with a flat entrance angle (180 degrees) with a capillary diameter of 2.0 mm and an aspect ratio (capillary length/capillary diameter) of 15. After equilibrating the samples at 190°C for 10 minutes, the piston is run at a constant piston speed of 0.265 mm/second. The standard test temperature is 190°C. The sample is drawn uniaxially to a set of accelerating nips located 100 mm below the die, with an acceleration of 2.4 mm/second$^2$. The tensile force is recorded as a function of the take-up speed of the nip rolls. Melt strength is reported as the plateau force (cN) before a strand breaks. The following conditions are used in the melt strength measurements: plunger speed = 0.265 mm/second; wheel acceleration = 2.4 mm/s$^2$; capillary diameter = 2.0 mm; capillary length = 30 mm; and barrel diameter = 12 mm.

2% Secant Modulus/Stress at Break

Tensile properties, including the secant modulus at 2% strain and the stress at break, are determined in the machine and cross directions according to ASTM D882.

Spencer Dart Impact Strength

The Spencer dart test is determined according to ASTM D3420, Procedure B.

Peel Force

Films are ultrasonically bonded to a nonwoven to form a laminate. The specimen size is 127 mm X 25.4 mm. Three specimens are measured per laminate. Peel force is determined by separating the film from a nonwoven substrate, and is a measure of the energy required to separate the layers. The film is placed in the movable grip of a CRE tensile testing machine (Instron) while the nonwoven substrate is placed in a stationary 180° plane. The films are peeled from the nonwoven substrate at a rate of about 304.8 mm/min.

Puncture Resistance

Puncture is measured on a tensile testing machine according to ASTM D5748, except for the following: square specimens are cut from a sheet to a size of 6 inches by 6 inches; the specimen is clamped in a 4 inch diameter circular specimen holder and a puncture probe is pushed into the center of the clamped film at a cross head speed of 10 inches/minute; the probe is a 0.5 inch diameter polished steel ball on a 0.25 inch support rod; there is a 7.7 inch maximum travel length to prevent damage to the test fixture; there is no gauge length - prior to testing, the probe is as close as possible to, but not touching, the specimen. A single thickness measurement is made in the center of the specimen. A total of five specimens are tested to determine an average puncture value.

Noise

Noise tester equipment includes an acoustic isolated box that contains a microphone MK 221 used to capture sound and a NC 10 Audio Acoustic Analyzer by Neutrix Cortex Instruments. The microphone is sensitive to a signal having a Frequency (Hz) of 20 Hz - 20,000 Hz. The microphone is located in the center of the acoustic box at 10 cm horizontally aligned with the film surface and 25 cm vertically aligned with the box top. The acoustic isolated box is made of lead with dimensions of 53 cm x 53 cm x 53 cm. Films are cut to a specimen size of 10 cm X 10 cm. The specimen is fixed to two holders, a first holder that is stationary and a second holder that is movable to provide a flexing motion of the film. The equipment is run in vacuum to obtain ground-noise readings that are subtracted from noise readings generated by each specimen. The data is collected on the 1/3 octave. Four different specimens are measured per film.

Softness

The "softness" or "hand" quality is considered to be the combination of resistance due to surface friction, flexibility, and compressibility of a fabric material. A Handle-O-Meter tester (manufactured by Thwing-Albert Instrument Co., West Berlin, N.J.) measures the above factors using a Linear Variable Differential Transformer (LVDT) to detect the resistance that a blade encounters when forcing a specimen of material into a slot of parallel edges. Samples are cut into 8 in x 8 in square specimens. The Handle-O-Meter slot width is set at 20 mm. Measurements are taken in each of four positions per specimen as required by the instrument manufacturer's test manual, and the four measurements are summed to give the total hand for a single specimen in grams-force. This averaged hand is then normalized to the specimen weight and volume. Samples having a lower resistance value are considered to have better softness.

EXAMPLES

The embodiments described herein may be further illustrated by the following nonlimiting examples.

Example 1

This example was conducted to demonstrate breathability of the film. Three layer films were made as outlined below. The films were produced on a three layer commercial cast line having a maximum line speed of 200 m/min, a melt temperature of 260°C, a die temp of 260°C, a die gap of 0.8 mils, and an air gap of 9 inches. The multilayer films have a basis weight of 14 gsm. The core layer comprises 70% of the overall film thickness. Each skin layer comprises 15% of the overall film thickness. Total film thickness is 1 mil.

The tested samples comprised two comparative samples (monolayers) and two multilayer coextruded samples. The comparative samples were monolayer films. The first comparative sample (Sample #1) contained 90 wt% DOWLEX® 2036 commercially available from Dow Chemicals and 10 wt% LDPE 722. The second comparative sample (Sample #2) was also a monolayer film and contained 55 wt% of the composition of Sample #1 with the remainder being calcium carbonate in an amount of 45 wt% based on the total weight of the monolayer.

The coextruded samples (the inventive samples - Sample #3 and #4) were 3-layer multilayer films and contained 45 wt% of calcium carbonate in each layer. Table 1 shows the ingredients for the Sample #'s 3 and 4. Each layer of the Sample # 3 and #4 contained 10 wt% of LDPE 722. This is not shown in the Table 1.

Table 1

| Sample ID | Layer A (first skin) (15% of total thickness) | Layer B (core layer) (70% of total thickness) | Layer C (second skin) (15% of total thickness) |
|---|---|---|---|
| Sample #3 | PP* (with 45 wt% $CaCO_3$) | DOWLEX® 2036 (with 45 wt% $CaCO_3$) | PP (with 45 wt% $CaCO_3$) |
| Sample #4 | PP (with 45 wt% $CaCO_3$) | ELITE® 5230 (with 45 wt% $CaCO_3$) | PP (with 45 wt% $CaCO_3$) |
| *PP = polypropylene | | | |

The samples were all ultrasonically bonded to a non-woven polypropylene laminate using HiQ Dialog. The ultrasonic device is a VE 20 MICROBOND CSI. The films detailed above were not subjected to stretching. Following the ultrasonic bonding they were subjected to a peel force test as detailed above. Figure 1 shows the peel test results.

From the Figure 1 it may be seen that sample that does not contain any filler (Sample #1-which contains only LDPE and Dowlex 2036) has a peel strength between 1.5 and 2 lbf/inch at a bonding force between 2000 and 2500 Newtons. The Sample #2 (which contains calcium carbonate at 45 wt% in addition to the LDPE and Dowlex 2036) shows a very low peel strength of less than 0.5 lbf/inch when bonded using a force between 2000 and 2500 Newtons. The inventive samples - Sample #3-which has the Dowlex core and Sample #4 - which has the Elite core both show an improved peel force strength of greater than or equal to 1 lbf/inch at a bonding force of 2000 to 2500 Newtons. This demonstrates that the use of polypropylene skin containing the filler produces a useful peel force strength of greater than 0.75 lbf/inch preferably greater than 1.0 lbf/inch and more preferably greater than 1.2 lbf/inch when a bonding force of 1500 Newtons or greater is used.

In summary, the use of a polypropylene skin with a filler produces a breathable multilayer film and also produces an improved peel force strength over a monolayer film that contains polyethylene.

While particular embodiments of the present invention have been illustrated and described, it would be obvious to those skilled in the art that various other changes and modifications can be made without departing from the scope of the claims.

## Claims

1. A multilayer film comprising:

    a core layer; and
    two skin layers; wherein the core layer is positioned between the two skin layers; wherein the core layer comprises a polyethylene polymer blend, the polyethylene polymer blend comprising at least 40%, by weight of the polyethylene polymer blend, of an ethylene-based polymer having a density of 0.900-0.940 g/cc and a melt index of 0.7-6 g/10 min, wherein the polyethylene polymer blend has an overall density of 0.910-0.950 g/cc and a melt index of 0.7-6 g/ 10 min, the melt index and density being measured according to the description, wherein the polyethylene polymer blend comprises from 0 to 15 %, by weight of the polyethylene polymer blend, of a low density polyethylene; and
    wherein each skin layer independently comprises a propylene-based polymer; wherein the propylene-based polymer comprises a propylene homopolymer, wherein each skin layer independently comprises at least 55 wt.%

of the propylene homopolymer,
wherein the skin layers are not non-wovens; and
wherein the multilayer film further comprises a filler, the filler being present in one or more layers of the multilayer film.

2. The film of claim 1, wherein the core layer and each skin layer contains the filler.

3. The film of claim 1, wherein the multilayer film is microporous.

4. The film of claim 1, wherein the core layer and the skin layer are elongated uniaxially or biaxially and where the film has a thickness of 0.005 to 0.038 mm (0.2 to 1.5 mils) and displays a water vapor transmission rate of greater than 500 g/m$^2$/24 hours, measured according to the description.

5. The film of claim 1, wherein the polypropylene polymer blend comprises 5 to 30 wt.% of the low density polyethylene and greater than 60 wt.% propylene-based polymer.

6. The film of claim 1, wherein the propylene homopolymer is isotactic, atactic or syndiotactic.

7. The film of claim 1, wherein the propylene-based polymer has a melt flow rate of about 2-50 g/10 min.

8. The film of claim 1, wherein the core layer comprises from 50% to 90% of the overall film thickness.

9. The film of claim 1, wherein the two skin layers have an equal thickness.

10. The film of claim 1, wherein the filler comprises a metal oxide, a metal hydroxide, or a combination comprising at least one of the foregoing.

11. The film of claim 1, wherein the filler is calcium carbonate present in an amount of 30 to 70 wt%, based on a total weight of the core layer or the total weight of each skin layer.

12. An ultrasonically bonded laminate comprising:

a multilayer film according to claim 1; and
a nonwoven substrate at least partially ultrasonically bonded to the multilayer film.

13. The laminate of claim 12, wherein the nonwoven substrate is made from a propylene-based material and wherein the laminate exhibits at least one of the following properties:

a peel force value of greater than 87.6 N/m (0.5 lbf/inch), measured according to the description; and
where the film has a thickness of 0.005 to 0.038 mm (0.2 to 1.5 mils) and displays a water vapor transmission rate of greater than 500 g/m$^2$/24 hours, measured according to the description.

14. A multilayer film comprising:

a core layer; wherein the core layer comprises a polyethylene polymer blend, the polyethylene polymer blend comprising at least 40%, by weight of the polyethylene polymer blend, of an ethylene-based polymer having a density of 0.900-0.940 g/cc and a melt index of 0.7-6 g/10 min, wherein the polyethylene polymer blend has an overall density of 0.910-0.950 g/cc and a melt index of about 0.7-6 g/ 10 min, wherein the polyethylene polymer blend comprises from 0 to 15 %, by weight of the polyethylene polymer blend, of a low density polyethylene;
a first layer that contacts the core layer; where the first layer comprises a propylene-based polymer; wherein the propylene-based polymer comprises a propylene homopolymer, wherein the first layer comprises at least 55 wt.% of the propylene homopolymer;
wherein the first layer is not non-woven, and
a nonwoven substrate at least partially ultrasonically bonded to the first layer on a surface that is opposed to a surface that contacts the core layer.

**Patentansprüche**

1. Mehrschichtfolie, umfassend:

    eine Kernschicht; und
    zwei Hautschichten; wobei die Kernschicht zwischen den beiden Hautschichten positioniert ist; wobei die Kernschicht eine Polyethylenpolymermischung umfasst, wobei die Polyethylenpolymermischung mindestens 40 Gew.-% der Polyethylenpolymermischung aus einem Polymer auf Ethylenbasis mit einer Dichte von 0,900 bis 0,940 g/cm$^3$ und einem Schmelzindex von 0,7 bis 6 g/10 min umfasst, wobei die Polyethylenpolymermischung eine Gesamtdichte von 0,910 bis 0,950 g/cm$^3$ und einen Schmelzindex von 0,7 bis 6 g/10 min aufweist, wobei Schmelzindex und Dichte gemäß der Beschreibung gemessen werden,
    wobei die Polyethylenpolymermischung 0 bis 15 Gew.-% der Polyethylenpolymermischung eines Polyethylens niedriger Dichte umfasst; und
    wobei jede Hautschicht unabhängig ein Polymer auf Propylenbasis umfasst; wobei das Polymer auf Propylenbasis ein Propylenhomopolymer umfasst, wobei jede Hautschicht unabhängig voneinander mindestens 55 Gew.-% des Propylenhomopolymers umfasst,
    wobei die Hautschichten keine Vliesstoffe sind; und
    wobei die Mehrschichtfolie ferner einen Füllstoff umfasst, wobei der Füllstoff in einer oder mehreren Schichten der Mehrschichtfolie vorhanden ist.

2. Folie nach Anspruch 1, wobei die Kernschicht und jede Hautschicht den Füllstoff enthalten.

3. Folie nach Anspruch 1, wobei die Mehrschichtfolie mikroporös ist.

4. Folie nach Anspruch 1, wobei die Kernschicht und die Hautschicht uniaxial oder biaxial gestreckt sind und wobei die Folie eine Dicke von 0,005 bis 0,038 mm (0,2 bis 1,5 mils) aufweist und eine Wasserdampfdurchlässigkeit von mehr als 500 g/m$^2$/24 Stunden aufweist, gemessen gemäß Beschreibung.

5. Folie nach Anspruch 1, wobei die Polypropylenpolymermischung 5 bis 30 Gew.-% Polyethylen niedriger Dichte und mehr als 60 Gew.-% Polymer auf Propylenbasis umfasst.

6. Folie nach Anspruch 1, wobei das Propylenhomopolymer isotaktisch, ataktisch oder syndiotaktisch ist.

7. Folie nach Anspruch 1, wobei das Polymer auf Propylenbasis einen Schmelzindex von etwa 2 bis 50 g/10 min aufweist.

8. Folie nach Anspruch 1, wobei die Kernschicht von etwa 50 % bis etwa 90 % der Gesamtfoliendicke ausmacht.

9. Folie nach Anspruch 1, wobei die zwei Hautschichten eine gleiche Dicke aufweisen.

10. Folie nach Anspruch 1, wobei der Füllstoff ein Metalloxid, ein Metallhydroxid oder eine Kombination aus mindestens einem der Vorstehenden umfasst.

11. Folie nach Anspruch 1, wobei der Füllstoff Calciumcarbonat ist, das in einer Menge von 30 bis 70 Gew.-%, bezogen auf das Gesamtgewicht der Kernschicht oder das Gesamtgewicht jeder Hautschicht, vorhanden ist.

12. Ultraschallgebundenes Laminat, umfassend:

    eine Mehrschichtfolie nach Anspruch 1; und
    ein Vliessubstrat, das an die Mehrschichtfolie mindestens teilweise ultraschallgebunden ist.

13. Laminat nach Anspruch 12, wobei das Vliessubstrat aus einem Material auf Propylenbasis hergestellt ist und wobei das Laminat mindestens eine der folgenden Eigenschaften aufweist:

    einen Schälkraftwert von mehr als 87,6 N/m (0-5 Ibf/inch), gemessen gemäß der Beschreibung; und|
    wobei die Folie eine Dicke von 0,005 bis 0,038 mm (0,2 bis 1,5 mils) aufweist und eine Wasserdampfdurchlässigkeit von mehr als 500 g/m$^2$/24 Stunden aufweist, gemessen gemäß Beschreibung.

**14.** Mehrschichtfolie, umfassend:

eine Kernschicht; wobei die Kernschicht eine Polyethylenpolymermischung umfasst, wobei die Polyethylenpolymermischung mindestens 40 Gew.-% der Polyethylenpolymermischung aus einem Polymer auf Ethylenbasis mit einer Dichte von 0,900 bis 0,940 g/cm$^3$ und einem Schmelzindex von 0,7 bis 6 g/10 Min. umfasst, wobei die Polyethylenpolymermischung eine Gesamtdichte von 0,910 bis 0,950 g/cm$^3$ und einen Schmelzindex von etwa 0,7 bis 6 g/10 Min. aufweist, wobei die Polyethylenpolymermischung 0 bis 15 Gew.-% der Polyethylenpolymermischung aus Polyethylen niedriger Dichte umfasst;
eine erste Schicht, die mit der Kernschicht in Kontakt steht; wobei die erste Schicht ein Polymer auf Propylenbasis umfasst; wobei das Polymer auf Propylenbasis ein Propylenhomopolymer umfasst, wobei die erste Schicht zu mindestens 55 Gew.-% das Propylenhomopolymer umfasst;
wobei die erste Schicht kein Vlies ist, und
ein Vliessubstrat, das mindestens teilweise ultraschallgebunden an die erste Schicht auf einer Oberfläche ist, die einer Oberfläche gegenüberliegt, die mit der Kernschicht in Kontakt steht.

## Revendications

**1.** Film multicouche comprenant :

une couche centrale ; et
deux couches superficielles ; dans lequel la couche centrale est positionnée entre les deux couches superficielles ; dans lequel la couche centrale comprend un mélange de polymère de polyéthylène, le mélange de polymère de polyéthylène comprenant au moins 40 %, en poids du mélange de polymère de polyéthylène, d'un polymère à base d'éthylène ayant une masse volumique de 0,900 à 0,940 g/cm$^3$ et un indice de fusion de 0,7 à 6 g/10 min, dans lequel le mélange de polymère de polyéthylène a une masse volumique globale de 0,910 à 0,950 g/cm$^3$ et un indice de fusion de 0,7 à 6 g/10 min, l'indice de fusion et la masse volumique étant mesurés conformément à la description,
dans lequel le mélange de polymère de polyéthylène comprend de 0 à 15 %, en poids du mélange de polymère de polyéthylène, d'un polyéthylène de faible masse volumique ; et
dans lequel chaque couche superficielle comprend indépendamment un polymère à base de propylène ; dans lequel le polymère à base de propylène comprend un homopolymère de propylène, dans lequel chaque couche superficielle comprend indépendamment au moins 55 % en poids de l'homopolymère de propylène,
dans lequel les couches superficielles ne sont pas des non-tissés ; et
dans lequel le film multicouche comprend en outre une charge, la charge étant présente dans une ou plusieurs couches du film multicouche.

**2.** Film selon la revendication 1, dans lequel la couche centrale et chaque couche superficielle contiennent la charge.

**3.** Film multicouche selon la revendication 1, dans lequel le film multicouche est microporeux.

**4.** Film selon la revendication 1, dans lequel la couche centrale et la couche superficielle sont allongées uniaxialement ou biaxialement et où le film a une épaisseur de 0,005 à 0,038 mm (0,2 à 1,5 mil) et affiche un taux de transmission de vapeur d'eau supérieur à 500 g/m$^2$/24 heures, mesuré conformément à la description.

**5.** Film selon la revendication 1, dans lequel le mélange de polymère de polypropylène comprend de 5 à 30 % en poids du polyéthylène de faible masse volumique et plus de 60 % en poids de polymère à base de propylène.

**6.** Film selon la revendication 1, dans lequel l'homopolymère de propylène est isotactique, atactique ou syndiotactique.

**7.** Composition selon la revendication 1, dans lequel le polymère à base d'éthylène a un indice de fusion d'environ 2 à 50 g/10 min.

**8.** Film selon la revendication 1, dans lequel la couche centrale constitue de 50 % à 90 % de l'épaisseur de film globale.

**9.** Film selon la revendication 1, dans lequel les deux couches superficielles ont une épaisseur égale.

**10.** Film selon la revendication 1, dans lequel la charge comprend un oxyde métallique, un hydroxyde métallique, ou une combinaison comprenant au moins l'un de ce qui précède.

**11.** Film selon la revendication 1, dans lequel la charge est du carbonate de calcium présent en une quantité de 30 à 70 % en poids, sur la base d'un poids total de la couche centrale ou du poids total de chaque couche superficielle.

**12.** Stratifié lié par ultrasons comprenant :

un film multicouche selon la revendication 1 ; et
un substrat non tissé lié au moins partiellement par ultrasons au film multicouche.

**13.** Stratifié selon la revendication 12, dans lequel le substrat non tissé est fabriqué à partir d'un matériau à base de propylène et dans lequel le stratifié présente au moins l'une des propriétés suivantes :

une valeur de force de pelage supérieure à 87,6 N/m (0,5 livre-force/pouce), mesurée conformément à la description ; et
où le film a une épaisseur de 0,005 à 0,038 mm (0,2 à 1,5 mil) et affiche un taux de transmission de vapeur d'eau supérieur à 500 g/m$^2$/24 heures, mesuré selon la description.

**14.** Film multicouche comprenant :

une couche centrale ; dans lequel la couche centrale comprend un mélange de polymère de polyéthylène, le mélange de polymère de polyéthylène comprenant au moins 40 %, en poids du mélange de polymère de polyéthylène, d'un polymère à base d'éthylène ayant une masse volumique de 0,900 à 0,940 g/cm$^3$ et un indice de fusion de 0,7 à 6 g/10 min, dans lequel le mélange de polymère de polyéthylène a une masse volumique globale de 0,910 à 0,950 g/cm$^3$ et un indice de fusion d'environ 0,7 à 6 g/10 min, dans lequel le mélange de polymère de polyéthylène comprend de 0 à 15 %, en poids du mélange de polymère de polyéthylène, d'un polyéthylène de faible masse volumique ;
une première couche qui est en contact avec la couche centrale ; où la première couche comprend un polymère à base de propylène ; dans lequel le polymère à base de propylène comprend un homopolymère de propylène, dans lequel la première couche comprend au moins 55 % en poids de l'homopolymère de propylène ;
dans lequel la première couche n'est pas non tissée, et
un substrat non tissé au moins partiellement lié par ultrasons à la première couche sur une surface qui est opposée à une surface qui est en contact avec la couche centrale.

Figure 1

EP 3 154 777 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2003106560 A1
- US 20081108268 A1
- US 5272236 A
- US 5278272 A
- US 5582923 A
- US 5733155 A
- US 3645992 A
- US 4076698 A
- US 3914342 A
- US 5854045 A
- WO 2005111291 A1
- US 4599392 A
- WO 2005023912 A
- WO 2007081548 A
- WO 1998004397 A

### Non-patent literature cited in the description

- **RANDALL**. *Rev. Macromol. Chem. Phys.*, vol. C29 (2,3), 285-297
- **ZIMM, B. H.** ; **STOCKMAYER, W. H.** *J. Chem. Phys.*, 1949, vol. 17, 1301
- **RUDIN A.** Modern Methods of Polymer Characterization. John Wiley & Sons, 1991, 103-112